# EUROPEAN PATENT APPLICATION

(11) **EP 2 335 692 A1**
(43) Date of publication of application: **22.06.2011**
(21) Application number: 10193173.1
(22) Date of filing: 30.11.2010
(51) Int. Cl.: A61K 31/02, A61K 31/075, A61K 31/695, A61K 31/80, A61P 27/02

(54) **Silicone liquid and fluorinated liquid for sequential use in the treatment of retinal tears and/or detachments**

(30) Priority: 04.12.2009 IT MI20092152
(71) Applicant: AL.CHI.MI.A. S.r.l., 35020 Ponte S. Nicolò (Padova) (IT)
(72) Inventor: Beccaro, Mauro, 35010, Padova (IT); Bettini, Enrico, 30032, VENEZIA (IT); Signori, Paolo, 37131, Verona (IT)
(74) Representative: Ponchiroli, Simone

(57) **Abstract**

A first liquid having a density lower than 1.00 g/cm³ and a second liquid having a density higher than 1.00 g/cm³ for sequential use in the surgical treatment of tears and/or detachments of the retina, where the said liquids are injected separately into the vitreous cavity after removal of the vitreous humour. In particular, is silicone liquid and is injected first and kept within the vitreous cavity for a determined period of time. Subsequently, the second liquid is injected, which is a fluorinated liquid, which, being miscible with the first liquid, forms a homogenous solution with the latter, with no phase separation and a density higher than 1.00 g/cm³. This solution exerts a tamponing action also on the lower portion of the retina, replacing the aqueous phase where PVR would otherwise tend to develop.

## Description

This invention relates to a first liquid having a density lower than 1.00 g/cm³ and a second liquid having a density higher than 1.00 g/cm³ for sequential use in the treatment of retinal tears and/or detachment.

The use of tamponing agents is well known for the treatment of retinal tears and/or detachments during vitreoretinal surgery. When injected into the vitreous cavity, these exert a hydrostatic pressure on the cavity walls to keep the retina in the correct position and favour the physiological processes of reattachment of the retina to the adjacent ocular components. The biocompatibility of these liquids allows to keep them within the ocular structure for a determined period time without causing damage to the structure itself.

Compounds belonging to two different classes, silicones and perfluorocarbons, have been proposed as tamponing liquids, each with different limitations and disadvantages. A third class consists of a mixture of the above products, so-called "heavy silicone oils" (e.g. the commercial product Densiron™ 68 by Fluoron, composed of a mixture of polydimethylsiloxane and perfluorohexyloctane). In all cases, however, these liquids are essentially immiscible with water.

The density of silicone oils (particularly polydimethylsiloxane) is too low, generally approximately 0.97 g/cm³ and thus below that of water; hence they tend to emerge from the aqueous medium which constitutes the vitreous humour. They are thus unable to guarantee sufficient pressure on the retina, and particularly the lower retina. In order to try to overcome this limitation, it was pursued to formulate fluorinated silicone compounds having a higher density than that of water. Nevertheless, these compounds proved unsuitable for the purpose, as they can cause considerable inflammatory responses, due to the difficulty of obtaining products that are sufficiently pure.

Perfluorocarbons have a high density (generally above 1.4 g/cm³) and a very low surface tension (below 25 mN/m), hence they enable complete relaxation and correct repositioning of the retina, thus favouring reattachment. Moreover, on a temporary basis, they are chemically inert and physiologically biocompatible, only if completely fluorinated. Indeed, the presence of -CHF-CF₂- may give rise to dehydrofluorination reactions with the formation of HF, resulting in the reduction of biocompatibility. Furthermore, once their activity as tamponing liquids has been completed during surgery, perfluorocarbons cannot be maintained in contact with the retina for a long time, since the high density may give rise to thinning of the lower retina over time. Hence, the use of perfluorocarbons is recommended exclusively during the intraoperative process. These must always be removed and replaced with a long-term tamponing liquid, such as a silicone oil.

In order to try and overcome the limitations deriving from the separate use of silicone liquids and perfluorocarbons, the combined use of these compounds has been proposed, as described in US patent 5.219.844. The combination of a perfluorocarbon liquid (particularly, perfluorophenanthrene C₁₂F₂₄) and a silicone oil, which are immiscible together and may be introduced separately or simultaneously into the vitreous cavity, allows to obtain a support pressure for the retina both in the lower portion, where the heavier perfluorocarbon is present, and in the upper portion where the lighter silicone oil tends to settle.

One of the most frequent causes of failure in retinal tear and/or detachment surgery is due to the onset of proliferative vitreoretinopathy (PVR). This consists of the proliferation of epithelial cells, glial cells and fibroblasts, which form contractile membranes within the vitreous cavity and on both sides of the retina, causing retinal detachment to reoccur.

Starting with the observation that cell proliferation occurs only in aqueous environments, the Applicant undertook to reduce as far as possible the presence of the aqueous phase on the inner surface of the vitreous cavity and at the same time achieve a tamponing effect that is as uniform as possible on both the upper and lower retina. In particular, the Applicant observed that the combined use of a silicone liquid and a perfluorinated liquid as suggested by US patent 5219844 is not capable of effectively contrasting the PVR, since, being immiscible together, the two liquids form two separate bubbles within the vitreous cavity, an upper one composed of the silicone liquid and a lower one composed of the perfluorocarbon. Between the inner wall of the vitreous cavity and the two bubbles a ring-shaped aqueous environment with cusp is formed where the PVR can form. The Applicant has now found that the above problem can be solved by using as tamponing liquids, in a combined and sequential way, a first liquid having a density lower than 1.00 g/cm³ and a second liquid having a density higher than 1.00 g/cm³. In particular, in its preferred embodiment, the first liquid is a silicone liquid or a mixture of a silicone liquid and a fluorinated liquid, while the second liquid is a fluorinated liquid or a mixture of a silicone liquid and a fluorinated liquid. The second liquid is also miscible with the first liquid to obtain a homogeneous solution. Both liquids are basically immiscible with water.

In accordance with this invention, after removal of the vitreous humour, only one of the two liquids is initially injected into the vitreous chamber. Depending on whether this has a density higher or lower than 1.00 g/cm³, this liquid acts as a tampon for the lower or upper portion, respectively, of the retina. After a determined period of time, generally 5-20 days, the other liquid is injected into the vitreous chamber, preferably inside the liquid bubble formed by the first injected liquid. At this point, after a certain amount of time determined according to circumstances, being miscible, the two liquids combine to form a homogeneous solution, that is, free of phase separation. In particular, the second liquid injected, which has a different density from the first injected liquid, is injected in such quantities to allow the density of the homogeneous solution to be respectively higher or lower than 1.00 g/cm³, if the first injected liquid has a density that is respectively lower or higher than 1.00 g/cm³. This way, following addition of the second injected liquid, the tamponing solution obtained moves from the lower portion of the retina to the upper portion of the retina, or vice versa depending on how its density varies, such that it also exercises a tamponing action on the portion of the retina on which the first injected liquid was not active, replacing the aqueous phase where PVR would otherwise tend to develop.

In particular, in a preferred form of use of the liquids that are the subject of this invention, after removal of the vitreous humour, the first liquid (e.g. a silicone liquid) is injected into the vitreous chamber, which acts as a tampon for the upper portion of the retina. After a determined period of time, generally 5-20 days, the second liquid (e.g. a fluorinated liquid) is injected into the vitreous chamber, which is miscible with the first liquid and dissolves into it to form a homogenous solution, with no phase separation, and being denser determines an increase in the mixture's density such that it also acts as a tampon on the lower portion, replacing the aqueous phase where PVR would otherwise tend to develop.

In a second form of use of the two liquids described in the previous paragraph, it is also possible to inject the second liquid first and the first liquid subsequently (in such quantities to bring the solution's density to below 1.00 g/cm³). This way, a tamponing action is achieved on the lower portion of the retina in the first stage and on the upper portion in the second stage.

In a first aspect, this invention thus relates to a first liquid having a density lower than 1.00 g/cm³ and a second liquid having a density higher than 1.00 g/cm³ (advantageously a silicone liquid and a fluorinated liquid, or a mixture of these) for sequential use in the surgical treatment of retinal tears and/or detachment as indicated in the annexed claims.

The aforementioned liquids enable implementation of a surgical method for the treatment of retinal tears and/or detachments, which involves the following sequence:
removing the vitreous humour from the vitreous cavity;
injecting into the vitreous cavity one of the two liquids (e.g. a silicone liquid) to act as a tampon on the upper or lower portion of the retina;
maintaining this liquid in the vitreous cavity for a determined period of time; after this time, injecting the other liquid into the vitreous cavity to achieve a limpid solution at body temperature to act as a tampon on the lower or upper portion, respectively, of the retina.

Introduction of the two liquids into the vitreous chamber takes place according to conventional techniques such as intravitreal injection. In particular, the subsequent introduction of the second injected liquid, which can be performed by intravitreal injection, does not require new surgical intervention.

Immediately after removal of the vitreous humour, a perfluorinated liquid can be injected into the vitreous chamber (e.g. a perfluorocarbon such as perfluorooctane or perfluorodecalin), which has the function of relaxing the retina. Before introduction of the first liquid or the second liquid in accordance with the invention, this perfluorinated liquid is eliminated completely, for example, by aspiration.

The first liquid is preferably a silicone liquid and has a viscosity of between 100 and 100,000 cS (centistokes), measured at 20°C. The density (measured at 20°C) of the silicone liquid is generally preferably between 0.760 and 0.990 g/cm³. This silicone liquid is preferably a silicone oil, and particularly polydimethylsiloxane.

The second liquid is preferably a fluorinated liquid composed of a partially fluorinated hydrocarbon compound, in case containing at least one ether group. Generally, the fluorinated liquid has a density (measured at 20°C) that is preferably between 1.01 and 1.99 g/cm³.

The second liquid is miscible with the first liquid at body temperature (conventionally 37°C), which means that the two liq uids form a homogeneous solution with no phase separation. Miscibility does not necessarily occur for all reciprocal ratios, but may be limited to composition intervals of varying width. The appropriate second liquid will then be selected according to its solubility in the first liquid and the intended density for the mixture of the two liquids, which must be lower or higher, respectively, than 1.00 g/cm³ depending on whether the first injected liquid has a respective density above or below 1.00 g/cm³. Generally, the solubility of the second liquid is equal to at least 5% in weight, and preferably a minimum of 20% in weight.

As already stated, in its preferred embodiments, the first liquid is a silicone liquid or a mixture of a silicone liquid and a fluorinated liquid, while the second liquid is a fluorinated liquid or a mixture of a silicone liquid and a fluorinated liquid.

Fluorinated liquids particularly suitable for the present invention may be chosen from fluoroalkyloxy alkanes with the formula (I):

R_{F}-R₁-O-R₂ (I)

where:
R_{F} is a linear or branched perfluoroalkyl group, having from 1 to 12, and preferably from 2 to 8, carbon atoms;
R₁ is a linear or branched non-fluorinated alkyl group, having from 1 to 6, and preferably from 2 to 4, carbon atoms;
R₂ is a linear or branched non-fluorinated alkyl group, having from 1 to 12, and preferably from 2 to 8, carbon atoms, in case containing at least one -O-ether bond along the chain.
R_{F} is preferably a linear perfluoroalkyl group with the formula CF₃(CF₂)ₙ-, where n is a integer from 1 to 11, more preferably from 2 to 7.
R₁ is preferably a linear alkyl group with the formula -(CH₂)ᵣ-, where r is a integer from 1 to 6, more preferably from 2 to 4.
R₂ is preferably a linear non-fluorinated alkyl group with the formula CH₃(CH₂)ₘ-, where m is a integer from 1 to 11, more preferably from 2 to 7. R₂ may contain at least one ether bond along the chain; for example R₂ may have the following formula:

   -(CH₂O)ₚ-(CH₂CH₂O)_{q}-R₃
where: R₃ is -CH₃ or -C₂H₅; p is zero or a integer between 1 and 4; q is a integer between 1 and 4; the -CH₂O- and -CH₂CH₂O- groups being distributed statistically along the chain.

Particularly preferred fluoroalkyloxy alkanes with formula (I) are: CF₃(CF₂)₅CH₂CH₂O(CH₂)₄CH₃, CF₃(CF₂)₅CH₂CH₂O(CH₂)₂CH₃, CF₃(CF₂)₃CH₂CH₂O(CH₂)₄CH₃, e CF₃(CF₂)₃CH₂CH₂O(CH₂)₂CH₃.

The fluoroalkyloxy alkanes with (I) preferably have a density that may vary from 1.2 to 1.5 g/cm³, measured at 20°C.

Further details on fluoroalkyloxy alkanes with formula (I) may be found in international patent application WO 2009/133575.

Another class of fluorinated liquids that can be used in accordance with the present invention is composed of semi-fluorinated alkanes with the formula:

R_{F}'R_{H} (II)

or:

R_{F}'R_{H}R_{F}' (III)

where R_{F}' is a linear or branched perfluoroalkyl group and R_{H} is a linear or branched saturated alkyl group, in which the linear semi-fluorinated alkanes have the formula:

F(CF₂)ₙ(CH₂)ₘH (IV)

or:

F(CF₂)ₙ(CH₂)ₘ(CF₂)ₙF (V)

while branched semi-fluorinated alkanes include: -FCX- or -CX₂- units within the R_{F}' groups, where X = C₂F₅, C₃F₇ or C₄F₉; and -HCY- or -CY₂- units within the R_{H} groups, where Y = C₂H₅, C₃H₇ or C₄H₉,
where the total number of carbon atoms in the R_{F}' groups is between 1 and 20, and the total number of carbon atoms in the R_{H} groups is between 3 and 20.

The semi-fluorinated alkanes with the formula (II) preferably have a density that may vary from 1.1 to 1.7 g/cm³, measured at 20°C.

Particularly preferred semi-fluorinated alkanes with formula (II) are: C₆F₁₃C₈H₁₇, C₄F₉C₅H₁₁ e C₂F₅C₈H₁₇.

Further details of semi-fluorinated alkanes with the formula (II) can be found in US patent 6.262126.

Another class of fluorinated liquids that can be used in accordance with the present invention is composed of partially fluorinated ethers with the formula:

Rf(CH₂)ₙO(CH₂)ₙRf (VI)

where Rf is a fluorinated, saturated, monovalent organic group C1-C4, and n is 3 or 4. Rf is preferably a polyfluoroalkyl group C1-C4 or a perfluoroalkyl group C1-C4.

The ethers with formula (VI) preferably have a density above 1 and below 1.6; the density is more preferably between 1.2 and 1.3.

A particularly preferred partially fluorinated ether with formula (VI) is decafluoro-di-n-pentyl ether C₂F₅CH₂CH₂CH₂OCH₂CH₂CH₂C₂F₅.

Further details on partially fluorinated ethers with formula (VI) can be found in patent application WO 2005/117850.

Lastly, another fluorinated liquid that can be used in accordance with the present invention is hydrogenated fluorinated olefin with the formula:

C₈F₁₇-CH₂-CH=CH-CH₂-CH(CH₃)₂

The quantities of the first liquid and the second liquid to be used in the method presented in this invention must be selected mainly according to the location of retinal damage and the desired tamponing effect. Generally, the ratio by weight between the first liquid and the second liquid may vary from 99:1 to 1:99 and preferably from 90:1 to 60:40.

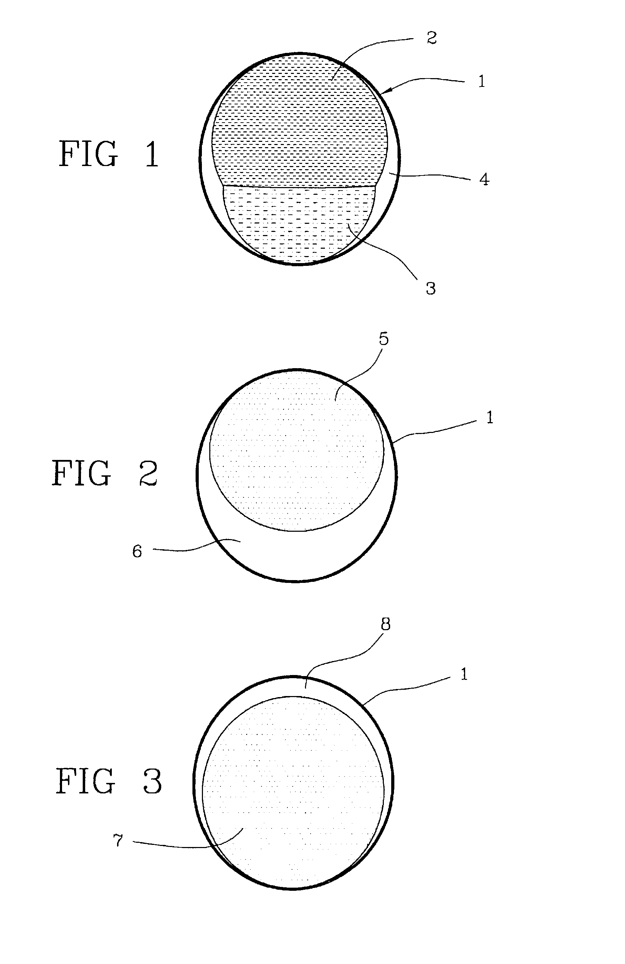

In a preferred embodiment of the invention, the first liquid and/or the second liquid are integrated with at least one anti-proliferation agent. This anti-proliferation agent is preferably added to the first injected liquid (ideally the first liquid in the preferred embodiment), in order to best guarantee continuance of the inhibitory action in the second stage of the method, when the upper portion of the chamber is no longer in contact with the tampon (see Fig. 3).

Possible anti-proliferation agents may be: 5-fluorouracil, triamcinolone, colchicine, taxol, daunomycin, adriamycin, carmustine, retinoic acid, alpha-tocopherol, or their mixtures.

In its preferred implementation form, the present invention will now be further illustrated with reference to the following figures in which:
Fig. 1 represents a diagram of the vitreous chamber where a silicone liquid and a perfluorinated liquid have been injected using the known technique (see US 5219844);
Fig. 2 represents a diagram of the vitreous chamber where the silicone liquid alone has been injected as per the present invention;
Fig. 3 represents a diagram of the vitreous chamber where the silicone liquid and subsequently the fluorinated miscible liquid have been injected according to the present invention.

With reference to Fig. 1, the vitreous chamber (1) contains an upper bubble (2) composed of the silicone liquid and a lower bubble consisting of the perfluorinated liquid (3). As they are immiscible together, the two liquids remain separate. As can be seen in Fig. 1, the presence of two separate bubbles generates a ring-shaped space with cusp (4) which holds an aqueous environment which favours the development of PVR.

Fig. 2 represents the vitreous chamber (1) where the silicone liquid has been injected, which has a density lower than that of water (that is, below 1.00 g/cm³ ), where it forms a bubble (5) which exerts pressure on the upper portion of the retina. In the portion of the wall that is in contact with the silicone liquid, the PVR is inhibited, since the silicone liquid replaces the aqueous phase.

In the lower part of the vitreous chamber (1) there remains a space (6) that is not occupied by the tamponing liquid, where the first proliferation phenomena are potentially triggered. For this reason, the time between injection of the silicone liquid and injection of the fluorinated liquid is relatively short, generally between 5 and 20 days, preferably between 7 and 10 days.

After introduction of the fluorinated liquid, which is completely miscible with the silicone liquid, the former combines with the latter to form a homogeneous solution which creates a single bubble (7) with an intermediate density between that of the silicone liquid and that of the fluorinated liquid but higher than 1.00 g/cm³, which tends to settle on the bottom of the vitreous chamber (1), thereby exerting pressure on the lower portion of the retina. Furthermore, this homogeneous solution replaces the aqueous phase present on the bottom, thus inhibiting any PVR phenomena that may have been activated during the first stage. Furthermore, it is important to note that the space (8) created in the upper portion of the vitreous chamber (1) does not create the risk of PVR, since the tamponing effect exerted by the silicone liquid in the first stage lasts over time. Indeed, during the first stage of treatment, the tamponing effect of the silicone liquid in the upper portion of the retina inhibits the reparative process which causes the formation of PVR, at the same time eliminating inflammation.

## Claims

1. A first liquid having a density lower than 1.00 g/cm³ and a second liquid having a density higher than of 1.00 g/cm³ for combined and sequential use in the treatment of tears and/or detachments of the retina, where
these liquids are basically immiscible with water; and
at body temperature, one liquid is miscible with the other liquid;
and where, in the treatment:
these liquids are injected separately into the vitreous cavity after removal of the vitreous humour,
the first liquid, or, respectively, the second liquid, is injected first and maintained within the vitreous cavity for a determined period of time,
after which, the second liquid, or respectively, the first liquid, is injected into the vitreous cavity to mix with the other liquid and create with the latter a homogeneous solution having a density that is higher or lower than 1.00 g/cm³ respectively, if the first injected liquid had a density that was lower or higher than 1.00 g/cm³.

2. The first and second liquid in claim 1, where the first liquid has a viscosity of between 100 and 100,000 cS (centistokes), measured at 20°C.

3. The first and second liquid in claim 1 or 2, where the first liquid has a density measured at 20°C of between 0.760 and 0.990 g/cm³.

4. The first and second liquid in any of the previous claims, where the second liquid has a density measured at 20°C of between 1.01 and 1.99 g/cm³.

5. The first and second liquid in any of the previous claims, where the second liquid has a solubility in the first liquid of at least 5% in weight, or at least 20% in weight.

6. The first and second liquid according to any of the above claims, where the first liquid is a silicone liquid or a mixture of a silicone liquid and a fluorinated liquid, and the second liquid is a fluorinated liquid or a mixture of a silicone liquid and a fluorinated liquid.

7. The first and second liquid in claim 6, where the fluorinated liquid is chosen from fluoroalkyloxy alkanes with the formula (I):
R_{F}-R₁-O-R₂ (I)
where:
R_{F} is a linear or branched perfluoroalkyl group, having from 1 to 12, and preferably from 2 to 8, carbon atoms;
R₁ is a linear or branched non-fluorinated alkyl group, having from 1 to 6, and preferably from 2 to 4, carbon atoms;
R₂ is a linear or branched non-fluorinated alkyl group, having from 1 to 12, and preferably from 2 to 8, carbon atoms, in case containing at least one -O-ether bond along the chain.

8. The first and second liquid in claim 7, where the fluoroalkyloxy alkane is chosen from: CF₃(CF₂)₅CH₂CH₂O(CH₂)₄CH₃, CF₃(CF₂)₅CH₂CH₂O(CH₂)₂CH₃, CF₃(CF₂)₃CH₂CH₂O(CH₂)₄CH₃, e CF₃(CF₂)₃CH₂CH₂O(CH₂)₂CH₃.

9. The first and second liquid in claim 6, where the fluorinated liquid is chosen from semi-fluorinated alkanes with the formula:
R_{F}'R_{H} (II)
or:
R_{F}'R_{H}R_{F}' (III)
where R_{F}' is a linear or branched perfluoroalkyl group and R_{H} is a linear or branched saturated alkyl group, where the linear semi-fluorinated alkanes have the formula:
F(CF₂)ₙ(CH₂)ₘH (IV)
or:
F(CF₂)ₙ(CH₂)ₘ(CF₂)ₙF (V)
while branched semi-fluorinated alkanes include: -FCX- or -CX₂- units within the R_{F}' groups, where X = C₂F₅, C₃F₇ or C₄F₉; and -HCY- or -CY₂- units within the R_{H} groups, where Y = C₂H₅, C₃H₇ or C₄H₉,
where the total number of carbon atoms in the R_{F}' groups is between 1 and 20, and the total number of carbon atoms in the R_{H} groups is between 3 and 20.

10. The first and second liquid in claim 9, where the semi-fluorinated alkane is chosen from: C₆F₁₃C₈H₁₇, C₄F₉C₅H₁₁ e C₂F₅C₈H₁₇.

11. The first and second liquid in claim 6, where the fluorinated liquid is chosen from partially fluorinated ethers with the formula:
Rf(CH₂)ₙO(CH₂)ₙRf (VI)
where Rf is a fluorinated, saturated, monovalent organic group C1-C4, and n is 3 or 4. Rf is preferably a polyfluoroalkyl group C1-C4 or a perfluoroalkyl group C1-C4.

12. The first and second liquid in claim 11, where the partially fluorinated ether with formula (VI) is decafluoro-di-n-pentyl ether C₂F₅CH₂CH₂CH₂OCH₂CH₂CH₂C₂F₅.

13. The first and second liquid in claim 6, where the fluorinated liquid is a hydrogenated fluorinated olefin with the formula C₈F₁₇-CH₂-CH=CH-CH₂-CH(CH₃)₂.

14. The first and second liquid in any of the previous claims, where the ratio by weight between the first liquid and the second liquid is between 99:1 and 1:99, or 90:1 and 60:40.

15. The first and second liquid in any of the previous claims, where at least one of the liquids, preferably the first liquid, is integrated with at least one anti-proliferation agent.

16. The first and second liquid in any of the previous claims, where the said homogeneous solution gives rise to a single bubble.

17. The first and second liquid in any of the previous claims, where the said homogeneous solution is limpid at body temperature.

18. The first and second liquid according to any of the above claims, where the first liquid is a silicone liquid and is injected first and the second liquid is a fluorinated liquid and is injected second.
